# EUROPEAN PATENT APPLICATION

(11) **EP 1 993 148 A1**
(43) Date of publication of application: **19.11.2008**
(21) Application number: 07714226.3
(22) Date of filing: 15.02.2007
(51) Int. Cl.: H01L 33/00, A61B 5/0245, A61B 5/1455, H01L 31/02

(54) **OPTICAL TRANSMISSION/RECEPTION DEVICE**

(30) Priority: 23.02.2006 JP 2006047249
(71) Applicant: HAMAMATSU PHOTONICS K. K., Hamamatsu-shi, Shizuoka 435-8558 (JP)
(72) Inventor: SHIBAYAMA, Katsumi, Hamamatsu-shi, Shizuoka 4358558 (JP)
(74) Representative: Frost, Alex John
(86) International application number: PCT/JP2007/052696
(87) International publication number: WO 2007/097240

(57) **Abstract**

An optical transmitting and receiving device 1 includes a main body portion 4 and has a base substrate 5 at a bottom-surface side of this main body 4. In a front surface 5a of this base substrate 5, formed is a recess-like first cavity 7 and second cavity 8, and an LD 9 is disposed in the first cavity 7, and a PD 12 is disposed in the second cavity 8. An LD terminal electrode 15 of the LD 9 is electrically connected, via a wire 28, to a base substrate wiring portion 19 formed on an insulating film 40 on the front surface 5a. Moreover, in a light shielding member 20 laminated on and fixed to the front surface 5a side of the base substrate 5, formed is a wire storing portion 29 to store the wire 28. Since these make it no longer necessary to provide a space to store the wire 28 in the first cavity 7, it becomes possible to downsize the first cavity 7. Accordingly, it becomes possible not only to downsize the base substrate 5 but also to downsize the optical transmitting and receiving device 1.

## Description

### Technical Field

The present invention relates to an optical transmitting and receiving device which is used mainly as a blood flow sensor for measuring a blood flow volume and the like.

### Background Art

As a conventional optical transmitting and receiving device, known is a blood flowmeter including a semiconductor substrate formed in its surface with recesses in which a light emitting element and a photodetecting element are disposed, respectively, and a cover substrate disposed on the surface of the semiconductor substrate and formed with a light shielding film (see Patent Document 1, for example). In this blood flowmeter, one terminal electrode of the light emitting element is electrically connected with an electrode formed on an inner surface of the recess via a wire.
Patent Document 1: Japanese Published Unexamined Patent Application No. 2004-229920

### Disclosure of the Invention

### Problem to be Solved by the Invention

However, such an optical transmitting and receiving device as described above has the following problem. That is, since one terminal electrode of the light emitting element is electrically connected with an electrode formed on an inner surface of the recess via a wire, it becomes necessary to provide a space to store the wire in the recess, so that the recess is increased in size by that space. Therefore, this hinders downsizing of the semiconductor substrate, and moreover, hinders downsizing of the optical transmitting and receiving device.

Hence, the present invention has been made in view of such circumstances, and it is an object to provide an optical transmitting and receiving device that can be downsized.

### Means for Solving the Problem

In order to attain the object described above, an optical transmitting and receiving device according to the present invention includes: a light emitting element for emitting light forward; a photodetecting element for receiving light irradiated from the front; a base substrate formed at a front surface thereof with a first recess in which the light emitting element is disposed and a second recess in which the photodetecting element is disposed; a light shielding member disposed at a front-surface side of the base substrate and formed with a first light passing hole through which light emitted by the light emitting element passes and a second light passing hole through which light to be received by the photodetecting element passes; and a light transmitting member which is disposed at a front-surface side of the light shielding member and through which light emitted by the light emitting element and light to be received by the photodetecting element are transmitted, wherein at least one of terminal electrodes of the light emitting element and terminal electrodes of the photodetecting element is electrically connected, via a wire, with a wiring formed on the front surface of the base substrate, and in the light shielding member, a wire storing portion to store the wire is formed.

Moreover, an optical transmitting and receiving device includes: a light emitting element for emitting light forward; a photodetecting element for receiving light irradiated from the front; a base substrate formed at a front surface thereof with a recess in which the light emitting element and the photodetecting element are disposed; a light shielding member disposed at a front-surface side of the base substrate and formed with a first light passing hole through which light emitted by the light emitting element passes and a second light passing hole through which light to be received by the photodetecting element passes; and a light transmitting member which is disposed at a front-surface side of the light shielding member and through which light emitted by the light emitting element and light to be received by the photodetecting element are transmitted, wherein at least one of terminal electrodes of the light emitting element and terminal electrodes of the photodetecting element is electrically connected, via a wire, with a wiring formed on the front surface of the base substrate, and in the light shielding member, a wire storing portion to store the wire is formed.

In these optical transmitting and receiving devices, at least one of terminal electrodes of the light emitting element and terminal electrodes of the photodetecting element is electrically connected, via a wire, with a wiring formed on the front surface of the base substrate, and in the light shielding member disposed at a front-surface side of the base substrate, a wire storing portion to store the wire is formed. Since this makes it no longer necessary to provide a space to store the wire in the recess, it becomes possible to downsize the recess. Accordingly, it becomes possible not only to downsize the base substrate but also to downsize the optical transmitting and receiving device.

In the optical transmitting and receiving device according to the present invention, it is preferable that the wire storing portion to store the wire electrically connected with at least one of the terminal electrodes of the light emitting element is a recess or a through-hole. In this case, the wire storing portion can be simply formed.

In the optical transmitting and receiving device according to the present invention, it is preferable that the wire storing portion to store the wire electrically connected with at least one of the terminal electrodes of the light emitting element is a through-hole, and this through-hole and the first light passing hole are continuously formed. In this case, the wire storing portion can be further simply formed.

In the optical transmitting and receiving device according to the present invention, it is preferable that the wire storing portion to store the wire electrically connected with at least one of the terminal electrodes of the photodetecting element is a recess. In this case, the wire storing portion can be simply formed, and light irradiated from the front can be prevented from being received by the wire storing portion via the wire storing portion.

### Effects of the Invention

According to the present invention, it becomes possible to downsize an optical transmitting and receiving device.

### Brief Description of the Drawings

[Fig. 1] A schematic perspective view of an optical transmitting and receiving device according to a first embodiment.
[Fig. 2] A schematic end view along a line II-II shown in Fig. 1.
[Fig. 3] A schematic exploded perspective view of a main body portion of the optical transmitting and receiving device shown in Fig. 1.
[Fig. 4] A schematic end view along a line IV-IV shown in Fig. 3.
[Fig. 5] A schematic end view, of a main body portion of an optical transmitting and receiving device according to a second embodiment, corresponding to Fig. 4.
[Fig. 6] A schematic end view, of an optical transmitting and receiving device according to a third embodiment, corresponding to Fig. 2.
[Fig. 7] A schematic end view along a line VII-VII shown in Fig. 6.
[Fig. 8] A schematic end view, of an optical transmitting and receiving device according to a fourth embodiment, corresponding to Fig. 2.
[Fig. 9] A schematic end view, of a main body portion of the optical transmitting and receiving device according to the fourth embodiment, corresponding to Fig. 4.

### Description of the Reference Numerals

1... optical transmitting and receiving device, 5 ... base substrate, 5a ... front surface, 7 ... first cavity (first recess), 8 ... second cavity (second recess), 9 ... LD (light emitting element), 12 ... PD (photodetecting element), 15, 16 ... LD terminal electrode, 17, 18 ... PD terminal electrode, 19 ... base substrate wiring portion (wiring), 20 ... light shielding member, 22 ... opening (first light passing hole), 23 ... pinhole (second light passing hole), 27 ... light transmitting member, 28, 31 ... wire, 29, 32 ... wire storing portion , 33 ... cavity (recess).

### Best Modes for Carrying Out the Invention

Hereinafter, preferred embodiments of the present invention will be described in detail with reference to the accompanying drawings. Also, the same or equivalent parts are denoted with the same reference numerals in the respective figures, and overlapping description will thus be omitted.

### [First Embodiment]

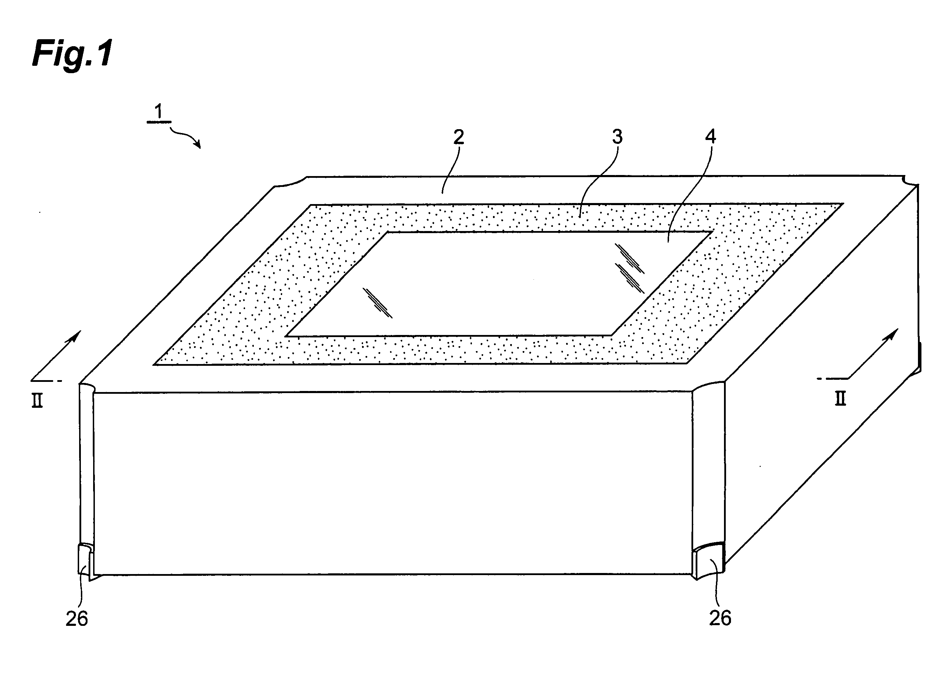

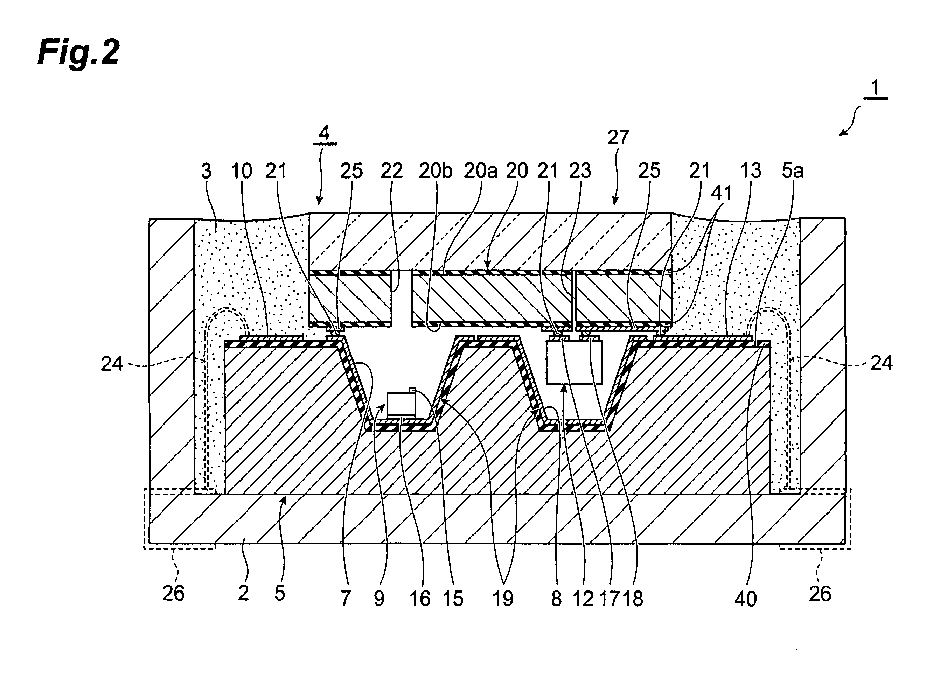

As shown in Fig. 1 and Fig. 2, an optical transmitting and receiving device 1 according to a first embodiment includes an enclosure 2 formed of, for example, a plastic material, in a rectangular-parallelepiped cup shape opened forward. In the enclosure 2, disposed is a main body portion 4 which incorporates an LD (light emitting element) 9 that emits light forward and a PD (photodetecting element) that receives light irradiated from the front, and around the main body portion 4 in the enclosure 2, filled is, for example, an insulating-coated, light shielding resin 3 made of a silicone resin containing a carbon filler. This optical transmitting and receiving device 1 is used as, for example, a blood flow sensor that detects a blood flow in a body tissue and measures a blood flow volume, a blood flow velocity, a pulse, and the like by means of scattered light scattered by irradiating light on the body tissue.

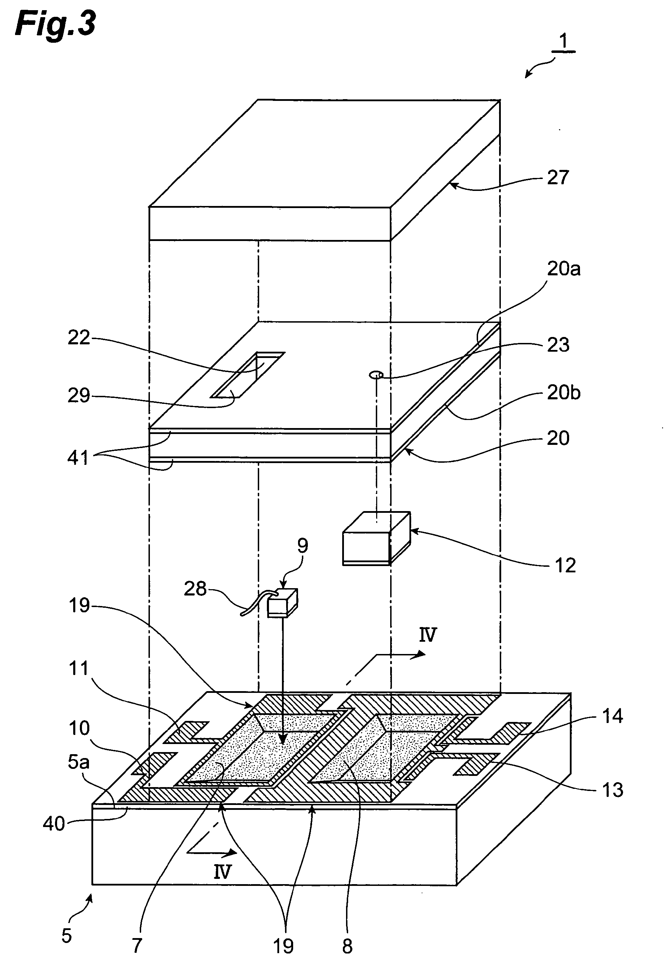

As shown in Fig. 2 and Fig. 3, the main body portion 4 has a base substrate 5 at a bottom-surface side in the enclosure 2. This base substrate 5 is formed of, for example, silicon being a semiconductor material, in a rectangular plate shape with a width of 2.8mm, a length of 6.0mm, and a thickness of 1.0mm, and in a front surface 5a of the base substrate 5, formed is a recess-like first cavity (first recess) 7 and second cavity (second recess) 8. The LD 9 is disposed in the first cavity 7, and is electrically connected with an LD anode 10 and an LD cathode 11 to be described later. Moreover, the PD 12 is disposed in the second cavity 8, and is electrically connected with a PD anode 13 and a PD cathode 14 to be described later.

Also, the cavity 7, 8 is formed by, for example, applying wet etching to a silicon substrate to be the base substrate 5. Concretely, the cavity 7, 8 is formed by providing, on the surface of a silicon substrate to be the base substrate 5, a mask formed of SiN or the like to demarcate the shape of the cavity 7, 8 and making an etchant act on an opening of this mask. After etching, the SiN mask is removed, and an insulating film 40 with a thickness of 1.5µm and made of, for example, SiO₂ is then formed on, at least, the surface of the cavity and the surface of the substrate by thermal oxidation.

The LD 9 uses, for example, a VCSEL (vertical-cavity surface-emitting laser) made of a compound semiconductor material with a thickness of 0.2mm, and this emits light having a wavelength of 850nm. In this LD 9, an LD terminal electrode 15 is provided at the front end face, and an LD terminal electrode 16 is provided at the rear end face. Moreover, the PD 12 uses, for example, a Si-PD or GaAs-PD made of a semiconductor material with a thickness of 0.3mm. In this LD 12, a PD terminal electrode 17 and a PD terminal electrode 18 are provided at the front end face.

Also, the material of the PD 12 is selected according to the wavelength of light to be emitted by the LD 9. For example, when the wavelength of light to be emitted by the LD 9 is 780nm, Si or GaAs is used as the material of the PD 12, and when the wavelength of light to be emitted by the LD 9 is 1.31µm, InGaAs is used as the material of the PD 12.

On the insulating film 40 on the front surface 5a of the base substrate 5 and the inner surface of the cavity 7, 8, formed by, for example, an A1 film, a Ti-Pt-Au laminated film, or a Cr-Pt-Au laminated film is a base substrate wiring portion (wiring) 19 with a predetermined pattern. This base substrate wiring portion 19 has an LD anode 10 and an LD cathode 11 on the first cavity 7 side and has a PD anode 13 and a PD cathode 14 on the second cavity 8 side. The LD anode 10, LD cathode 11, PD anode 13, and PD cathode 14 are, as shown in Fig. 1 and Fig. 2, electrically connected, via wires 24, with electrodes 26 formed at four corners in the bottom of the enclosure 2, in a manner extracting the electrodes from the front surface to the rear surface thereof. And, to the base substrate wiring portion 19 formed on the insulating film 40 on the bottom surface of the first cavity 7, the LD terminal electrode 16 of the LD 9 is electrically connected via, for example, a solder, a conductive resin, or the like.

Returning to Fig. 2 and Fig. 3, on the insulating film 40 on the front surface 5a of the base substrate 5, laminated is a light shielding member 20 formed of, for example, a silicon substrate coated with an insulating film 41 made of SiO₂ in a rectangular plate shape with a thickness of 0.15mm to 0.30mm, and this is fixed by bump bonding 21. Examples of the material for the bump bonding 21 include Au, Ni, Cu, AuSn, and SnAg-based solders. Here, it is preferable to pave a part between the first cavity 7 and the second cavity 8 with bump bonding 21 so that light emitted from the LD 9 does not reach the PD 12 through a gap between the base substrate 5 and the light shielding member 20. Alternatively, in this part, a light shielding portion may be formed by applying or filling a light shielding material.

In the light shielding member 20, formed is an opening (first light passing hole) 22 provided at a position corresponding to the first cavity 7 and a pinhole (second light passing hole) 23 provided at a position corresponding to the second cavity 8. The opening 22 guides light emitted from the LD 9 to the outside. Moreover, the pinhole 23 guides scattered light from the outside to the PD 12 and also prevents ambient light and unnecessary light from the outside from entering the PD 12. Also, the opening 22 and the pinhole 23 are formed by, for example, applying dry etching to a silicon substrate to be the light shielding member 20, and the pinhole 23 has a high aspect ratio.

Also, the pinhole 23 is formed at a position corresponding to a photodetecting surface of the PD 12, and the diameter thereof is provided as, for example, 30µm to 90µm. This is because noise due to ambient light and unnecessary light from the outside to be sensed by the PD 12 is increased when the diameter of the pinhole 23 is larger than 90µm, while when the diameter of the pinhole 23 is smaller than 30µm, light to be received by the PD 12 is reduced, so that an output from the PD 12 is lowered.

On the insulating film 41 on a rear surface 20b of the light shielding member 20, a light shielding member wiring portion 25 is formed with a predetermined pattern, and this is electrically connected with the base substrate wiring portion 19 by bump bonding 21. At a part corresponding to the second cavity 8 in the light shielding member wiring portion 25, the PD terminal electrode 17, 18 of the PD 12 is electrically connected (so-called, flip-chip bonding) by bump bonding 21. Thereby, the PD 12 is electrically connected with each of the PD anode 13 and PD cathode 14.

Moreover, on the insulating film 41 on a front surface 20a of the light shielding member 20, laminated is a light transmitting member 27 formed of, for example, alkali-containing borosilicate glass in a rectangular plate shape with a thickness of 0.3mm so that light emitted from the LD 9 and scattered light from the outside are transmitted therethrough, and this is fixed by a resin. This light transmitting member 27 enhances mechanical strength of the light shielding member 20 and also seals the first cavity 7 and the second cavity 8 of the base substrate 5 into a package. Also, since the light transmitting member 27 is fixed to the light shielding member 20, the coefficient of thermal expansion of the light transmitting member 27 and the coefficient of thermal expansion of the light shielding member 20 are set almost equal. Alternatively, the light shielding member 20 and the light transmitting member 27 may be fixed by anodic bonding. When anodic bonding is performed, the insulating film 41 is no longer necessary on the light transmitting member 27 side.

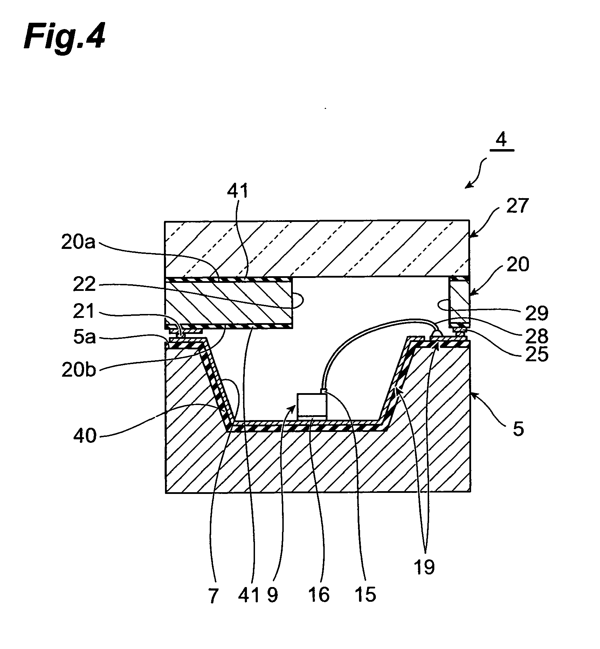

Meanwhile, in the optical transmitting and receiving device 1, as shown in Fig. 4, the LD terminal electrode 15 of the LD 9 is electrically connected, via a wire 28, to the base substrate wiring portion 19 formed on the insulating film 40 on the front surface 5a of the base substrate 5. Moreover, in the light shielding member 20 laminated on and fixed to the front surface 5a side of the base substrate 5, formed is a wire storing portion 29 to store the wire 28. Since these make it no longer necessary to provide a space to store the wire 28 in the first cavity 7, it becomes possible to downsize the first cavity 7. Accordingly, it becomes possible not only to downsize the base substrate 5 but also to downsize the optical transmitting and receiving device 1.

Further, in the light shielding member 20, the wire storing portion 29 to store the wire 28 is a through-hole, and this through-hole and the opening 22 are continuously formed. Adopting such a construction allows simply forming the wire storing portion 29 along with the opening 22.

When, for example, information concerning a fluid substance in a body such as a blood flow volume and the like in a body tissue is measured by use of the optical transmitting and receiving device 1 constructed as in the above, voltage is supplied to the LD anode 10 and the LD cathode 11 of the base substrate wiring portion 19 so as to emit light from LD 9 disposed in the first cavity 7 of the base substrate 5. This light passes through the opening 22 formed in the light shielding member 20, is transmitted through the light transmitting member 27 to be emitted to the outside, and then advances in the body tissue to be, for example, scattered by blood flow components. A part of the scattered light thus scattered advances in a direction opposite the forward direction of the light emitted from the LD 9, is transmitted through the light transmitting member 27, passes through the pinhole 23 formed in the light shielding member 20, and reaches the PD 12 disposed in the second cavity 8 of the base substrate 5. Then, a voltage signal in response to the reached light is obtained from the PD anode 13 and the PD cathode 14 of the base substrate wiring portion 19. Here, since the scattered light contains an interference component between scattered light from a stationary body tissue and scattered light that has received a Doppler shift from red blood cells moving in the capillaries of a body tissue, a frequency analysis of the voltage signal obtained by the PD 12 enables blood flow measurements etc.

### [Second Embodiment]

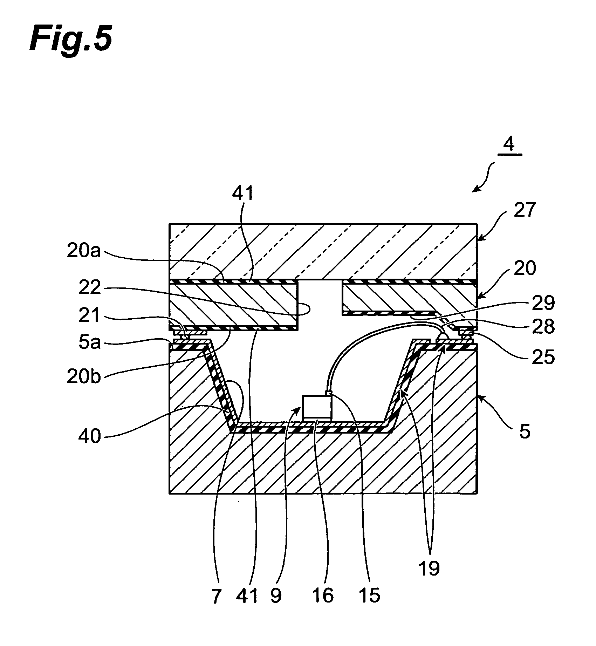

The optical transmitting and receiving device 1 according to a second embodiment differs in the shape of the wire storing portion 29 from the optical transmitting and receiving device 1 according to the first embodiment. More specifically, in the optical transmitting and receiving device 1 according to the second embodiment, as shown in Fig. 5, the wire storing portion 29 is provided as a recess formed on the rear surface 20b of the light shielding member 20, and is formed continuously with the opening 22.

The optical transmitting and receiving device 1 according to the second embodiment constructed as such also provides the same operations and effects as those of the optical transmitting and receiving device 1 according to the first embodiment.

### [Third Embodiment]

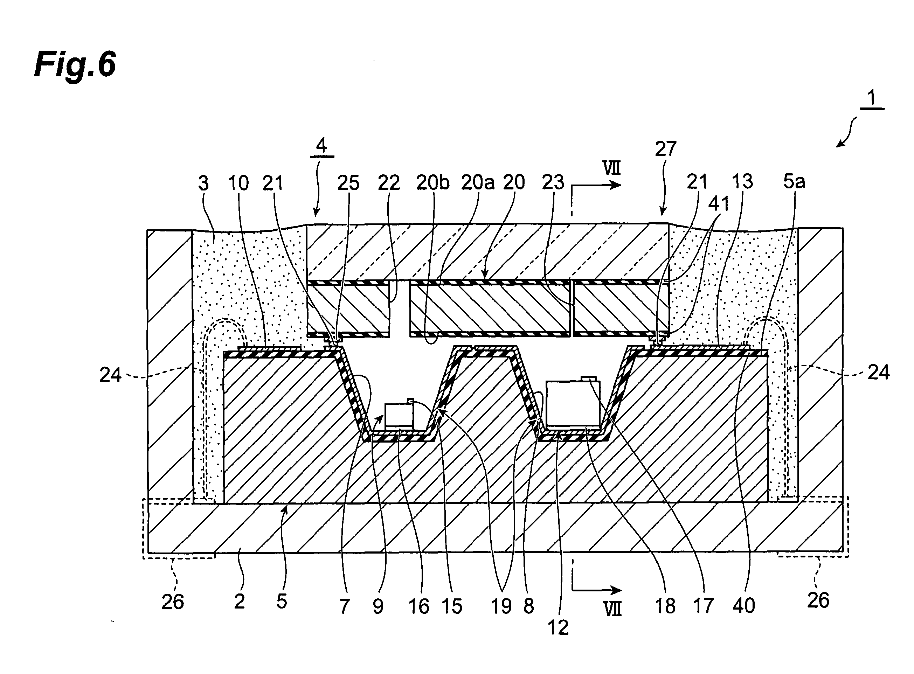

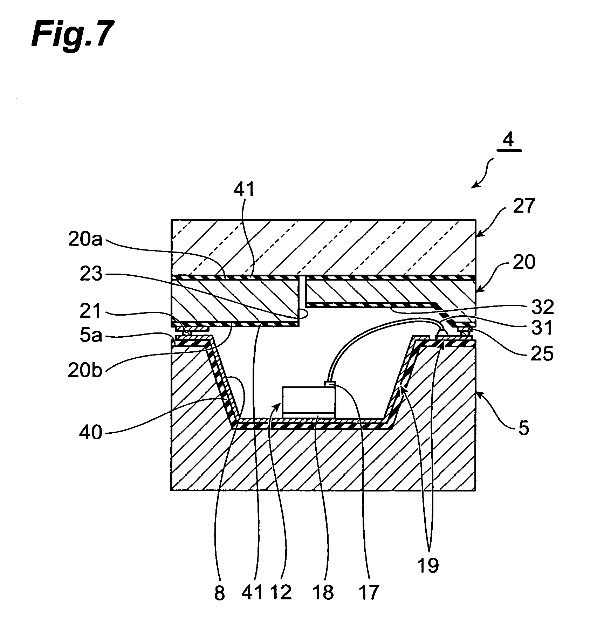

The optical transmitting and receiving device 1 according to a third embodiment differs in the construction of the PD 12 from the optical transmitting and receiving device 1 according to the first embodiment. More specifically, in the optical transmitting and receiving device 1 according to the third embodiment, as shown in Fig. 6 and Fig. 7, the PD terminal electrode 17 is provided at the front end face of the PD 12 and the PD terminal electrode 18 is provided at the rear end face of the PD 12. And, the PD terminal electrode 17 is electrically connected, via a wire 31, with the base substrate wiring portion 19 formed on the insulating film 40 on the front surface 5a of the base substrate 5, and the PD terminal electrode 18 is electrically connected with the base substrate wiring portion 19 formed on the insulating film 40 on the bottom surface of the second cavity 8. On the rear surface 20b of the light shielding member 20, formed as a recess is a wire storing portion 32 to store the wire 31, and the wire storing portion 32 is formed continuously with the pinhole 23. Also, in consideration of ambient light and unnecessary light that enter the PD 12, the length of the pinhole 23 is provided as 0.1mm or more.

The optical transmitting and receiving device 1 according to the third embodiment constructed as such also provides the same operations and effects as those of the optical transmitting and receiving device 1 according to the first embodiment.

### [Fourth Embodiment]

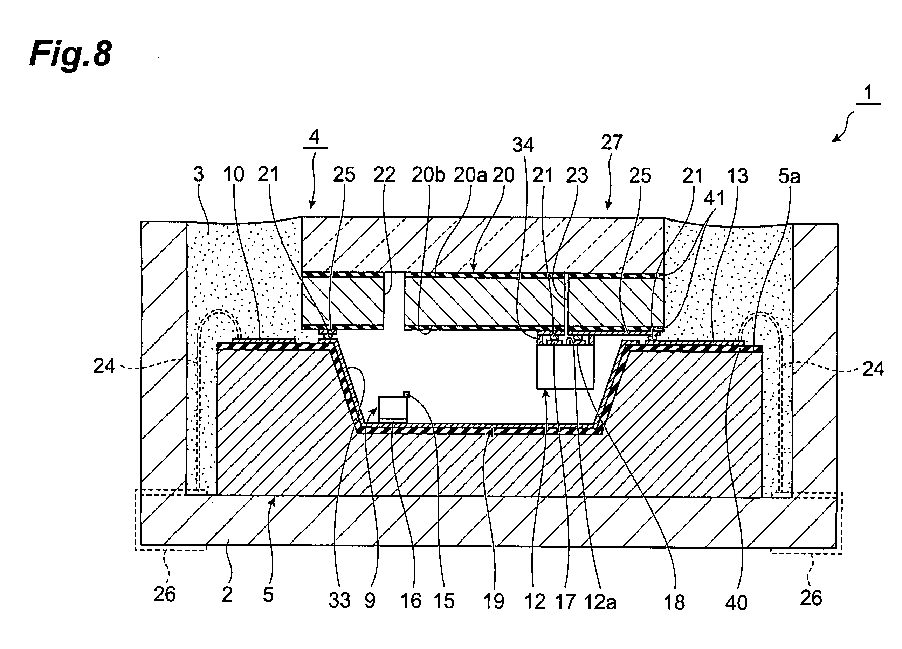

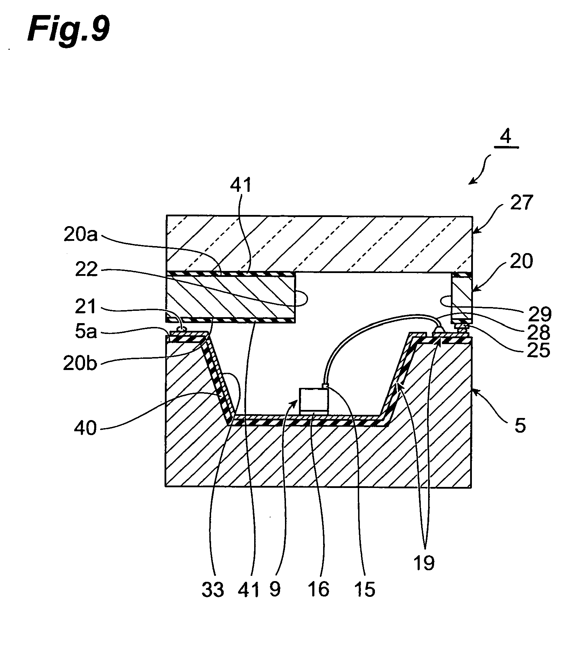

The optical transmitting and receiving device 1 according to the fourth embodiment differs in the shape of the base substrate 5 from the optical transmitting and receiving device 1 according to the first embodiment. More specifically, in the optical transmitting and receiving device 1 according to the fourth embodiment, as shown in Fig. 8 and Fig. 9, a cavity (recess) 33 in which the LD 9 and the PD 12 are disposed is formed in the front surface 5a of the base substrate 5. Between the light shielding member 20 and the PD 12, a light shielding portion 34 made of a light shielding resin is provided so as to surround a photodetecting surface 12a of the PD 12. This prevents ambient light and unnecessary light from entering the PD 12 due to light emission or the like of the LD 9.

The optical transmitting and receiving device 1 according to the fourth embodiment constructed as such also provides the same operations and effects as those of the optical transmitting and receiving device 1 according to the first embodiment.

In the above, although preferred embodiments of the present invention have been described, the present invention is not limited to the abovementioned embodiments.

For example, an optical transmitting and receiving device may be constructed such that a wire electrically connected with the LD terminal electrode of an LD is stored in a wire storing portion which is a recess formed in the rear surface of a light shielding member and a wire electrically connected with the PD terminal electrode of a PD is stored in a wire storing portion which is a recess formed in the rear surface of a light shielding member. Alternatively, an optical transmitting and receiving device may be constructed such that an LD formed at the front end face with an anode-side LD terminal electrode and a cathode-side LD terminal electrode is electrically connected by flip-chip bonding with a light shielding member wiring portion formed on an insulating film on the rear surface of a light shielding member and a wire electrically connected with the PD terminal electrode of a PD is stored in a wire storing portion which is a recess formed in the rear surface of the light shielding member.

Further, when the wire storing portion is a through-hole, the through-hole may be formed discontinuously with the opening (that is, independently of the opening). Moreover, when the wire storing portion is a recess, the recess may be formed discontinuously with the opening or pinhole (that is, independently of the opening or pinhole).

### Industrial Applicability

According to the present invention, it becomes possible to downsize an optical transmitting and receiving device.

## Claims

1. An optical transmitting and receiving device comprising:
a light emitting element for emitting light forward;
a photodetecting element for receiving light irradiated from the front;
a base substrate formed at a front surface thereof with a first recess in which the light emitting element is disposed and a second recess in which the photodetecting element is disposed;
a light shielding member disposed at a front-surface side of the base substrate and formed with a first light passing hole through which light emitted by the light emitting element passes and a second light passing hole through which light to be received by the photodetecting element passes; and
a light transmitting member which is disposed at a front-surface side of the light shielding member and through which light emitted by the light emitting element and light to be received by the photodetecting element are transmitted, wherein
at least one of terminal electrodes of the light emitting element and terminal electrodes of the photodetecting element is electrically connected, via a wire, with a wiring formed on the front surface of the base substrate, and
in the light shielding member, a wire storing portion to store the wire is formed.

2. An optical transmitting and receiving device comprising:
a light emitting element for emitting light forward;
a photodetecting element for receiving light irradiated from the front;
a base substrate formed at a front surface thereof with a recess in which the light emitting element and the photodetecting element are disposed;
a light shielding member disposed at a front-surface side of the base substrate and formed with a first light passing hole through which light emitted by the light emitting element passes and a second light passing hole through which light to be received by the photodetecting element passes; and
a light transmitting member which is disposed at a front-surface side of the light shielding member and through which light emitted by the light emitting element and light to be received by the photodetecting element are transmitted, wherein
at least one of terminal electrodes of the light emitting element and terminal electrodes of the photodetecting element is electrically connected, via a wire, with a wiring formed on the front surface of the base substrate, and
in the light shielding member, a wire storing portion to store the wire is formed.

3. The optical transmitting and receiving device according to Claim 1 or 2, wherein the wire storing portion to store the wire electrically connected with at least one of the terminal electrodes of the light emitting element is a recess or a through-hole.

4. The optical transmitting and receiving device according to Claim 1 or 2, wherein the wire storing portion to store the wire electrically connected with at least one of the terminal electrodes of the light emitting element is a through-hole, and this through-hole and the first light passing hole are continuously formed.

5. The optical transmitting and receiving device according to Claim 1 or 2, wherein the wire storing portion to store the wire electrically connected with at least one of the terminal electrodes of the photodetecting element is a recess.
